Europäisches Patentamt

(19) European Patent Office

Office européen des brevets

(11) Publication number: **0 245 547**
**B1**

# EUROPEAN PATENT SPECIFICATION

(45) Date of publication of the patent specification: **22.08.90**

(51) Int. Cl.⁵: **A61N 1/36**

(21) Application number: **86303600.0**

(22) Date of filing: **12.05.86**

(54) Electronic control system for controlling pelvic viscera via neuro-electrical stimulation.

(43) Date of publication of application:
**19.11.87 Bulletin 87/47**

(45) Publication of the grant of the patent:
**22.08.90 Bulletin 90/34**

(84) Designated Contracting States:
**AT BE CH DE FR GB IT LI LU NL SE**

(56) References cited:
US-A- 3 870 051
US-A- 3 888 261

IEEE ENGINEERING IN MEDICINE AND BIOLOGY, vol. 2, no. 2, June 1983, pages 31-36, IEEE, New York, US; R.A. SCHMIDT et al.: "Neural prostheses and bladder control"
WIRELESS WORLD, vol. 90, no. 1576, January 1984, pages 61-64, Sheepen Place, Oichester, GB; T. IVALL: "Radio activated implant for bladder control"

(73) Proprietor: **THE REGENTS OF THE UNIVERSITY OF CALIFORNIA, 2199 Addison Street, Berkeley California 94720(US)**

(72) Inventor: **Tanagho, Emil A., 43 San Marino Drive, San Rafael California 94720(US)**
Inventor: **Lue, Tom F., 407 Juanita Avenue, Milbrae California 94030(US)**
Inventor: **Schmidt, Richard A., 317 Parnassus Avenue, San Francisco California 94117(US)**
Inventor: **Gleason, Curtis A., 721 Charleston Court, Palo Alto California 94303(US)**

(74) Representative: **Loven, Keith James et al, Brookes & Martin High Holborn House 52/54 High Holborn, London WC1V 6SE(GB)**

ACTORUM AG

## Description

### Technical Field

This invention relates generally to an electronic control system for controlling bodily functions and, more particularly, to the utilization of one or more electrodes on selected nerve bundles and control means for applying pulse trains to the electrode(s) to control, regulate or treat such functions.

### Background Art

Various medical patients exhibit involuntary control over their bladder and/or bowel. Although vesicostomy or an artificial sphincter implanted around the urethra are commonly used to provide partial control over the evacuation function of the bladder and to control continence, these solutions have drawbacks well known to those skilled in the medical profession and related arts. Other patients who achieve a modicum of control over their bladder functions are equally in need of a system to rehabilitate their nerve and muscle dysfunctions. Similar problems arise in respect to involuntary bowel control.

The physiology of the bladder and bowel is closely linked to the urethral muscle physiology of the pelvic floor (levator ani muscle) and its related urethral and anal sphincters. For the bladder to store urine and for the bowel to serve as a reservoir for feces, two opposite, but complementary, behaviors are found. In particular, the bladder and rectum must relax and the urethral and anal sphincters must remain contracted. The reverse is true during evacuation of either urine or feces, i.e., the urethral or anal sphincter will relax along with the pelvic floor, and subsequently the bladder and rectum will contract.

The sequence will reverse once voiding and defecation is completed, i.e., the sphincters and pelvic floor muscles will revert to their tonic closure states and the bladder and rectum will revert to their storage states. This behavior has been demonstrated by simultaneous manometric (or EMG/pressure) recordings of this bladder/rectum, urethral/anal behavior during filling and emptying of the bladder. This sequence of events is well-established and is accepted universally. US-A 3 870 051 discloses a control system for stimulating the sphincter and bladder by applying electrical pulse trains simultaneously to the sacral ventral soot fibres governing the activity of the bladder and the sphincter muscles, with all pulses in a pulse train being either of high or low intensities.

### Disclosure of Invention

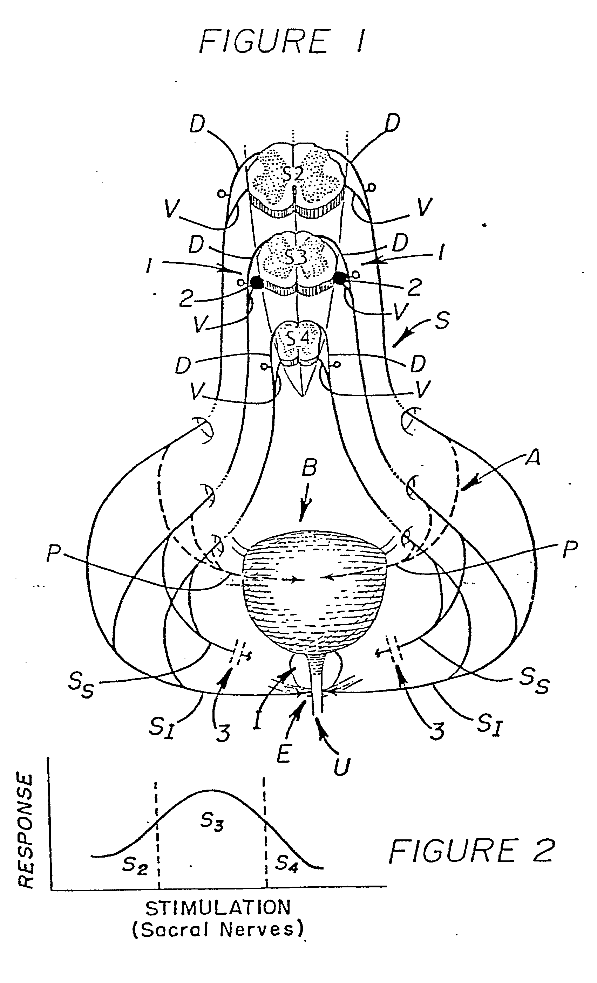

An electronic control system is disclosed for controlling bladder or bowel evacuation (Figures 1-11), for modulating the symptoms resulting from a loss of coordination between normally synchronized functions of visceral organs (Chart I), and for treating incontinence by increasing sphincter tonus (Chart II).

The present invention provides apparatus for controlling the functions of a bladder or bowel and its associated external sphincter in an anatomical system of a selected human, as defined in claim 1.

The term "controlling", "modulating" and "treating" as used herein not only include the selective control, modulation or treatment of a particular organ on a continuous basis, but further include isolated or periodic stimulation of the organ for diagnostic or rehabilitation purposes, e.g., neuromodulation of muscular behavior to rehabilitate muscular dysfunction in the pelvic floor without stimulating the pelvic nerve controlling the bladder's detrusor muscle. The term "organ" as used herein broadly means an independent part of the human body that performs a special function or functions, including visceral organs, such as the bladder, bowel and colon, and associated sphincters, cuffs and muscles.

The electronic control system includes electrode means adapted to be positioned on selected nerve bundles for electrically stimulating the nerve bundles. A control means is adapted to sequentially apply pulse trains to the electrode means sequentially to control the function of one organ or a number of organs simultaneously.

### Brief Description of the Drawings

Other advantages and objects of this invention will become apparent from the following description and accompanying drawings wherein:

Figure 1 schematically illustrates the pelvic plexus region in a human, including the nervous system for controlling bladder evacuation and related functions, and further illustrates a first operative procedure for controlling such functions by use of the electronic control system shown in Figures 12-17;

Figure 2 schematically illustrates a stimulation-response curve of bladder contraction in response to stimulation of the S2, S3 and S4 sacral nerves;

Figures 3 and 4 are views similar to Figure 1, but illustrate additional operative procedures for controlling bladder evacuation and related functions;

Figure 5 schematically illustrates the percutaneous implantation of an electrode adjacent to the S3 sacral nerve through the dorsum for the purpose of selectively stimulating such nerve;

Figures 6-11 are views similar to Figure 1, but illustrate additional operative procedures for controlling bladder evacuation and related functions;

Figure 12 illustrates a micturition control system adapted for use in conjunction with an operative procedure for controlling bladder and/or bowel evacuation and related functions;

Figure 13 schematically illustrates a typical electronic circuit for use in an implantable receiver of the Figure 12 micturition control system;

Figure 14 diagrammatically illustrates electronic signals and their timed relationship for the Figure 12 micturition control system;

Figure 15 illustrates an electrode arrangement, including pairs of electrodes attached to separate nerve bundles and adapted for use with the Figure 12 control system;

Figure 16 is a view similar to Figure 15, but illustrates a multiplicity of active electrode contacts on single electrodes;

Figure 17 diagrammatically illustrates electrical impulses in their timed relationship for the electrode arrangements illustrated in Figures 15 and 16; and

Figure 18 is a view similar to Figure 1, but illustrates the positioning of electrodes on various nerve bundles to effect desired results, as reflected in Charts I and II set forth hereinafter.


## DESCRIPTION (FIGURES 1-11)

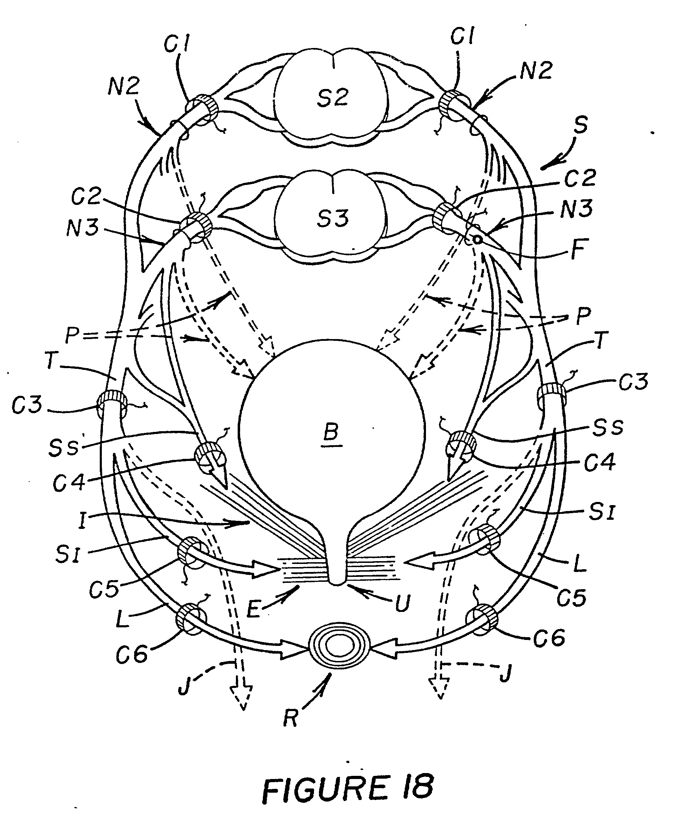

Figures 1 and 18 schematically illustrates the pelvic plexus region of a human, including the nervous system for controlling bladder and bowel evacuation and related functions. The nervous system includes a somatic nerve system of fibers (or nerve bundles) S and an autonomic nerve system of nerve bundles A, finding their immediate origin at sacral segments S2, S3 and S4 of the spinal cord and sacrum, i.e., the triangular bone positioned below the lumbar vertebrae and comprising five fused sacral vertebrae that are wedged dorsally between the two hip bones. As illustrated in Figure 2, the main nerve supply to the detrusor muscle of a bladder B emanates primarily from sacral segment S3, a lesser amount from sacral segment S2, and a still lesser amount from sacral segment S4, i.e., "response" refers to bladder response.

The electronic control system described in this application is adapted for controlling the bladder or bowel and related functions, modulating symptoms resulting from a loss of coordination between the normally synchronized functions of the bladder and bowel and their associated sphincters and treating incontinence by increasing sphincter tonus. Either permanent surgical implantation or temporary percutaneous implantation for nerve stimulation purposes is applicable. Also, electrodes can be implanted either unilaterally or bilaterally.

As further illustrated in Figure 1, the main nerve supply emanating from each sacral segment S2, S3 and S4 comprises two components or roots, namely, a dorsal root D and a ventral root V. The dorsal root is primarily sensory to transmit sensation to the spinal cord whereas the ventral root is primarily motor to transmit motor impulses from the spinal cord to bladder B and associated sphincter. Although illustrated as being separated, the dorsal and ventral roots for each nerve are, in fact, normally joined together and their fibres or bundles mixed to progress as a single trunk.

Bundles of the nerve trunk are divided into somatic nerve bundles S that connect to voluntary muscles and autonomic nerve bundles A that connect to visceral organs, such as bladder B. Dorsal root D can be separated from ventral root V since only stimulation of the motor nerve bundles of a particular ventral root are contemplated in many procedures. In this manner, the motor nerve bundles can be stimulated without inducing pain and without generating impulses along the sensory passageway.

Somatic nerves S and autonomic nerves A can also be separated from each other. For example, in a particular procedure wherein it is desirable to only drive muscles controlled by the somatic nerve, the somatic nerve can be solely stimulated. Should it prove desirable to control the muscles of only a visceral organ, such as the detrusor muscle of bladder B, the autonomic nerve bundles could be stimulated. Stimulation of the entire nerve trunk would function to stimulate each of the dorsal, ventral, somatic and autonomic nerve bundles.

However, such stimulation may prove undesirable for certain patients since uncoordinated action would ensue, e.g., bladder B and external sphincter E would each contract and no effective response to stimulation would be realized. Thus, the ability to isolate the dorsal and ventral roots from each other and to further isolate the autonomic nerves from the somatic nerves enables a practitioner to alleviate pain and to simultaneously achieve specific responses of the controlled organ or organs.

For example, responses obtained with pre-operative evaluation of responses to stimulation recorded urodynamically could indicate that the S2 sacral nerve constitutes the main motor supply to external sphincter E, whereas the S3 sacral nerve constitutes the main motor supply to bladder B. Thus, the S3 sacral nerve would be utilized to control the detrusor muscle and thus the contracting function of bladder B alone, whereas the S2 sacral nerve would be utilized to control the muscles controlling the continence function of external sphincter E. Studies have shown that in certain patients, only the nerves on one side of the sacrum provide the main motor control over a particular organ, i.e., unilateral control rather than bilateral control. Pre-operative testing of a particular patient will determine which variation

3

will provide the best choice for a subsequent operative procedure. The ability of this invention to isolate various components of the various nerves, with the combined ability to test a patient intraoperatively and record responses, has enabled the applicants herein to isolate and selectively stimulate the particular nerve bundles that will effect the specific function or functions required.

Figure 1 and 3-11 illustrate various combinations of operative procedures for effecting the desired neurostimulation for specific case studies (male or female) wherein simultaneous control for coordinating the synchronized functions of a bladder or bowel and its associated sphincter is effected. For example, a quadriplegic who has suffered a neck injury and damage to his spinal cord will normally require an operative procedure wherein control of bladder B and external sphincter E are of the utmost importance. In addition, the quadriplegic may suffer uncontrolled bowel evacuation, for example, which can be concurrently controlled when bladder control is effected by such operative procedure. In addition, it may prove desirable to modulate other voiding dysfunctions that may occur as a result of one or more of a multitude of other neurological reasons.

Thus, specific operative procedures herein described can be combined with one or more of the other procedures described herein, as dictated by pre-operative evaluation of responses to stimulation recorded urodynamically. For example, when a particular procedure (e.g., electrode implant, nerve separation, sectioning, etc.) is described as being performed bilaterally, clinical testing may indicate that in certain other patients, a unilateral procedure (or combination of bilateral and unilateral) is necessary (and vice versa). Likewise, the specific steps or procedures utilized in one operative procedure (Figures 1 and 3-11) may be utilized in combination with one or more steps utilized in other operative procedures, as will be appreciated by those skilled in the arts relating hereto.

Although the operative procedures herein described are primarily useful and applicable to coordinated control of bladder and bowel and their associated sphincters, similar procedures hereinafter more fully described are applicable to the control of other organs, including the colon, associated sphincters and cuffs and to the elimination or suppression of spastic detrusor activity, spastic urethral and pelvic floor activity and spastic anal sphincter. In all of the described operative procedures, it is assumed that pre-operative evaluation of response to stimulation has been recorded urodynamically to precisely locate the nerves requiring separation, neurostimulation and/or isolation, such as by sectioning.

## OPERATIVE PROCEDURE FOR CONTROLLING EVACUATION (FIGURE 1)

Although the following operative procedure shown in Figures 1 and 3-11 are primarily discussed in connection with the simultaneous control of the coordinated and synchronized functions of a bladder and its sphincter, they are also applicable to the control of a bowel and its sphincter.

Figure 1 illustrates an operative procedure whereby continence and evacuation of bladder B is closely controlled in a particular patient, such as a quadriplegic. The particular operative procedure utilized will depend upon a patient's ability to respond to electrical stimuli at strategic locations on his or her nervous system in the pelvic plexus region. For example, it is assumed in the Figure 1 operative procedure that the patient is unable to self-control his or her bladder functions and that such locations have been evaluated pre-operatively.

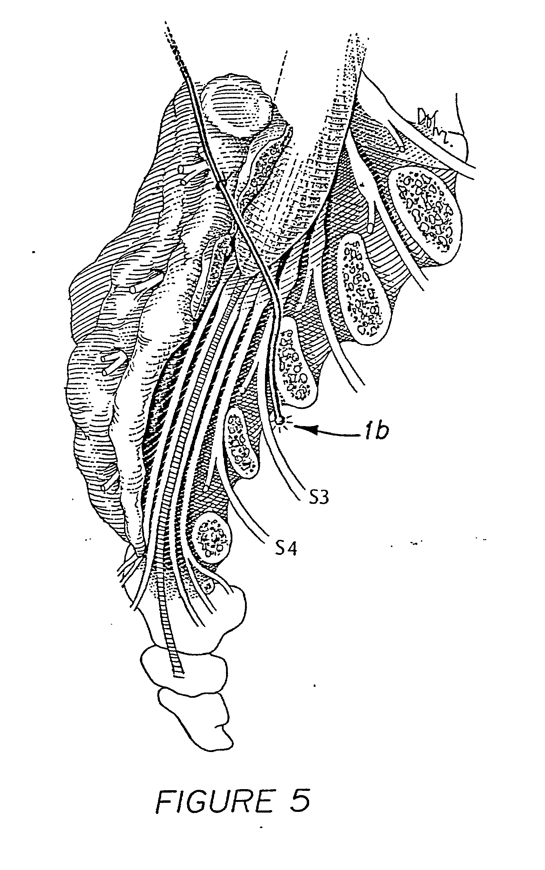

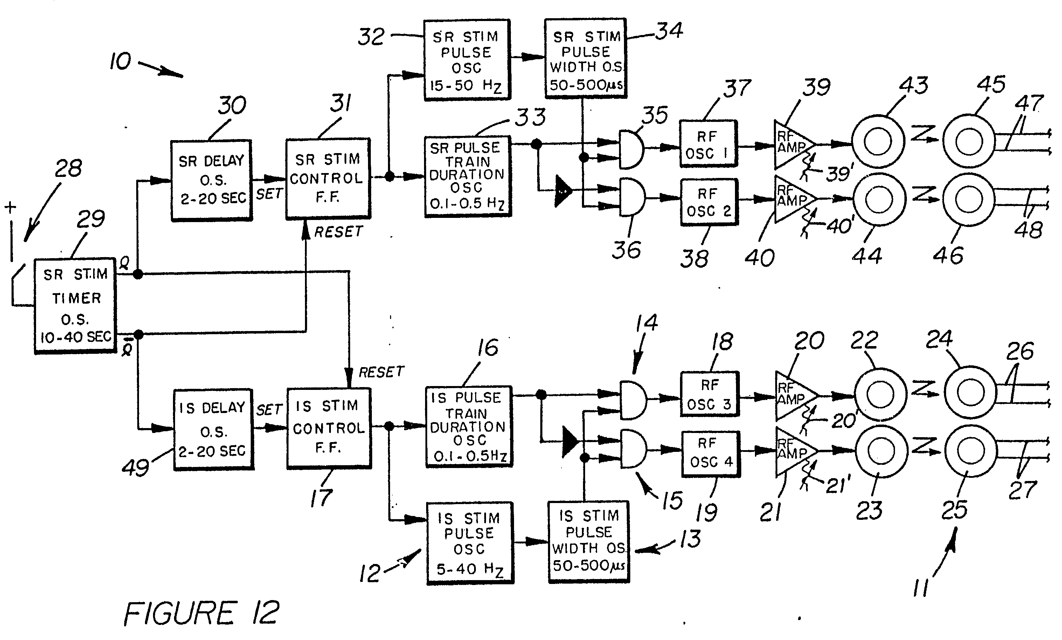

As shown in Figure 1, after the anatomical location of the S3 sacral nerve is identified, such as by the percutaneous insertion and electrical energization of an electrode placed at least in close proximity to such nerve, as illustrated in Figure 5, the dorsal (sensory) root D and ventral (motor) root V are surgically separated bilaterally on each side of sacral segment S3. An electrode 2 is then attached by sutures or otherwise implanted on each ventral root V for purposes of external excitation and stimulation, as hereinafter described with reference to the micturition control system illustrated in Figure 12.

After bilateral implantation of electrodes 2 on ventral components or roots V, each superior somatic nerve $S_s$ is sectioned at 3 bilaterally to eliminate any increase in the resistance normally provided by the levator ani muscles at least partially surrounding external sphincter E and controlled by superior somatic nerve $S_s$. Superior somatic innervation ($S_s$) is commonly described in anatomy books (e.g., CIBA or Gray's) as part of the innervation to the levator ani muscles, whereas inferior somatic innervation ($S_I$) is classically described as the pudendal nerve in Alcock's Canal. It should be noted that an internal sphincter I will normally open automatically when the bladder contracts and thus requires no artificial control.

The operative procedure in Figure 1 was preceded by identification of the S3 sacral nerve and confirmation that it controlled bladder and related functions by use of intraoperative stimulation and urodynamic recordings. Minimum requirements to effect bladder evacuation without sacrificing continence, i.e., the ability to retain contents of the bladder until conditions are proper for urination, were assumed to be confirmed. The subsequent bilateral separation of ventral root V from dorsal root D and the bilateral implantation of electrode 2 on the ventral root was found to minimize risk of pain or other undesirable reflexoenic response. Outlet resistance through urethra U was insured by sectioning superior somatic nerve $S_s$ at 3 whereby the various nerves controlling outlet resistance at external sphincter E were totally isolated, i.e., isolation of motor supply to the levator ani muscle.

Pre-operative electrostimulation was achieved by the use of a bipolar probe for stimulating the various nerve bundles. A nerve stimulator was then used to deliver a DC square wave for stimulation purposes. The nerve stimulator may be of the type manufactured by Grass Medical Instruments of Quincy, Massachusetts, U.S.A., under Model No. S-44. The electrodes are of the standard type. For example, each electrode may constitute a bipolar cuff electrode having an inside diameter approximating 3-5 mm. and provided with 1 mm. by 2 mm. platinum contacts having a 3 mm. separation placed outside each other about the periphery of ventral nerve root V. This type of electrode is manufactured by Avery Laboratories, Inc. under Model No. 390.

As described more fully hereinafter by reference to Figures 12-17, suitable receivers in the form of implantable silastic-coated units containing an antenna coil, adapted to receive RF (radio frequency) pulses from an external transmitter, are implanted subcutaneously in the patient to transmit pulses to the electrodes to control bladder evacuation in a controlled manner.

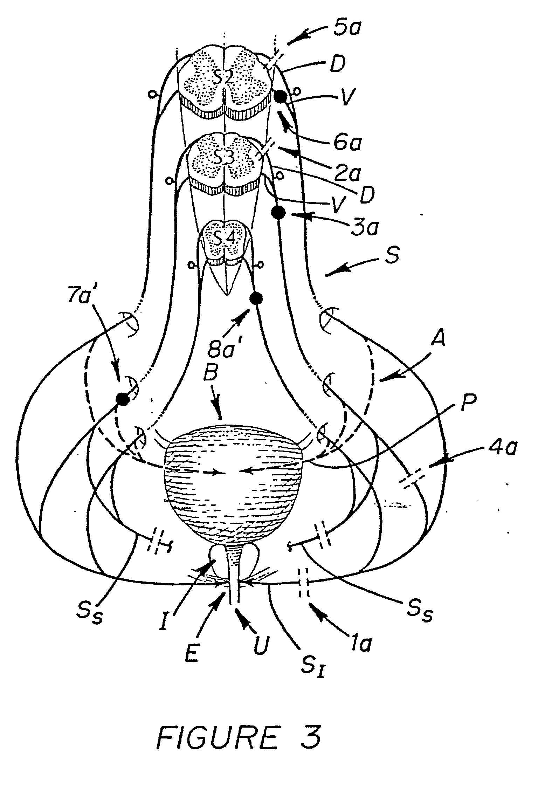

OPTIONAL OPERATIVE PROCEDURE FOR CONTROLLING EVACUATION (FIGURE 3)

Figure 3 illustrates optional variations to the Figure 1 operative procedure which will potentially enhance bladder evacuation. After the various critical nerves for controlling bladder evacuation have been identified by intraoperative stimulation and urodynamic recordings, each of the S2, S3 and S4 sacral nerves are separated to isolate the respective ventral and dorsal roots thereof. Pudendal or inferior somatic nerve $S_I$ is then sectioned unilaterally to isolate external sphincter E on one side. Dorsal root D of the S3 sacral nerve is then sectioned at 2a to thus isolate the sensory function thereof. Although illustrated as being performed unilaterally, and as stated above, in certain applications it may prove desirable to perform such sectioning bilaterally.

An electrode 3a is then implanted on the entire S3 sacral nerve unilaterally, with or without dorsal rhizotomies at other sacral levels. The S3 sacral nerve is then sectioned at 4a unilaterally (or bilaterally), downstream of pelvic nerve P to isolate this nerve's contribution to inferior somatic nerve $S_I$. It should be noted that electrode 3a is thus positioned on the S3 sacral nerve to stimulate the detrusor muscles of bladder B, via pelvic nerve P.

After appropriate separation of the dorsal and ventral roots of the S2 sacral nerve, the dorsal root is sectioned at 5a unilaterally (or bilaterally) and an electrode 6a is suitably implanted on the ventral root V of the S2 sacral nerve. It should be noted that superior somatic nerve $S_s$ is preferably sectioned bilaterally, as described above in reference to the Figure 1 operative procedure, to eliminate any additional increase in resistance from contraction of the levator ani muscle when the bladder is contracting for evacuation purposes.

The above options will also tend to eliminate or minimize a response in the pelvic floor sphincter which would otherwise prevent low resistance voiding of the bladder synchronous with stimulation. Thes optional variations address the possibility that excessive residual sphincter activity remains with stimulation after the Figure 1 operative procedure has been attempted. Sphincter response may be reflexly produced which suggests the need for dorsal sectioning at 2a and 5a in Figure 3, or directly produced to suggest sectioning 1a of inferior somatic $S_I$, unilaterally or bilaterally. The above steps must, of course, be carefully evaluated prior to the selected operative procedure so as not to compromise continence or the contraction of the bladder or bowel or nerves controlling the erection process.

Additional optional procedures may include percutaneous implantation of an electrode 7a' on sacral nerve S3 and/or S4, upstream of the point whereat the autonomic nerve roots forming pelvic nerve P separate from the respective sacral nerve proper, to aid in bladder contraction through the pelvic nerve. A further option contemplates implantation of a cuff electrode 8a' around sacral nerve S4, either unilaterally, as shown, or bilaterally, to assist in the control of bladder evacuation. It should be understood that above sectioning steps 2a and 5a, as well as the implantation of electrodes 3a, 6a and 8a', require laminectomy, i.e., incision of the posterior arch of the vertebrae.

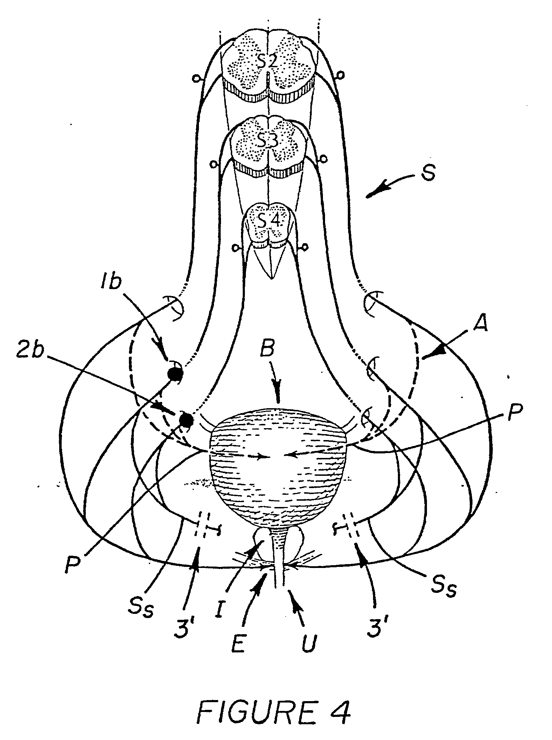

NON-LAMINECTOMY PROCEDURE FOR CONTROLLING VISCERAL ORGANS (FIGURES 4 AND 5)

Figure 4 illustrates an operative procedure wherein an electrode 1b is implanted onto the S3 sacral nerve through a sacral foramen without excising the posterior arch of the vertebrae. A second electrode 2b may be implanted in a like manner on the S4 sacral nerve, either in addition to or in lieu of electrode 1b. These electrode implants may be effected unilaterally, as illustrated, or bilaterally, depending on the pre-operative test results. Optionally, superior somatic nerve $S_s$ is sectioned at 3', either unilaterally or bilaterally as illustrated in Figure 4.

The system thus effected by the Figure 4 operative procedure will normally provide means for selectively eliminating or suppressing spastic detrusor activity, spastic urethral and pelvic floor activity and spastic anal sphincter. Such system may further suppress or enhance erection.

Figure 5 illustrates the percutaneous implantation of electrode 1b through the dorsum and the sacral forament of sacral segment S3 for the purpose of selectively stimulating the S3 sacral nerve. After the appropriate depth and location of the S3 nerve has been verified by electrostimulation and recorded urodynamically, electrode 1b can be inserted through the hollow spinal needle used for such stimulation with the wire lead connected to the electrode being suitably sutured in place, as shown, for attachment to a receiver (not shown), as will be described more fully hereinafter. This percutaneous method can also be used to temporarily implant an electrode on any one or more of the sacral nerves for testing purposes, i.e., to record activity in the bladder in response to stimulation of one or more of the nerves by the electrodes to thus determine which nerve or nerves are controlling the bladder functions. This procedure can be conducted unilaterally or bilaterally.

For example, electrode 1b could be percutaneously placed on the S3 sacral nerve with the external extremity of the wire attached to the electrode then being taped to the skin, along with a receiver connected thereto. The patient could then resume his day-to-day lifestyle and be allowed to stimulate the nerve or nerves artificially via a transmitter compatible with the receiver. If the response is positive and complete relief is achieved, the electrode or electrodes could be permanently implanted or temporarily implanted for the purpose of correcting any dysfunction by "retraining" the nerve and associated muscles. Should little or no improvement result, the same procedure could be followed to accurately ascertain which nerve or nerves require stimulation. Thus, this invention contemplates not only the implantation of one or more electrodes in the sacral nervous system for controlling evacuation of a visceral organ or the like, but also contemplates use of such electrodes and procedures to rehabilitate muscle dysfunction by neuromodulation of muscular behavior, as described more fully hereinafter with reference to Chart II.

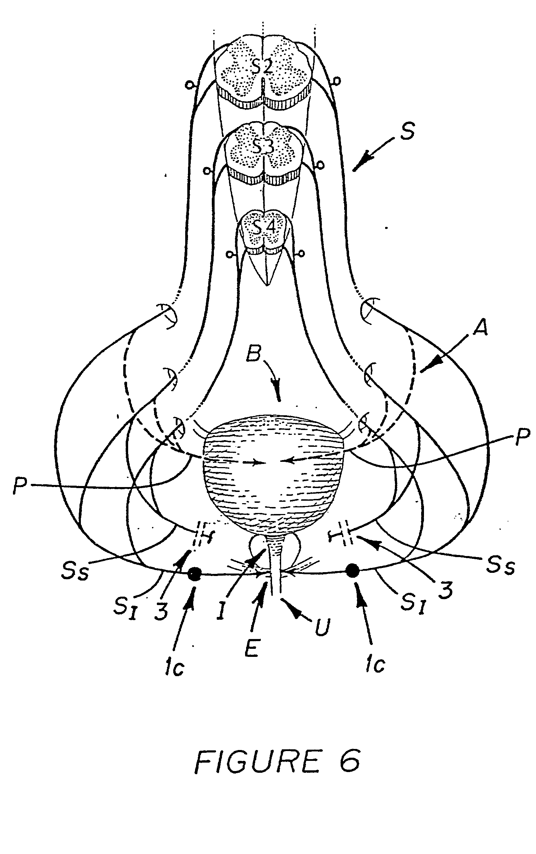

ADDITIONAL OPTIONAL OPERATIVE PROCEDURES (FIGS. 6-11)

Figure 6 illustrates an optional variation for controlling evacuation of bladder B. In particular, superior somatic nerve Ss is sectioned at 3, either unilaterally or bilaterally, as shown. In addition, electrodes 1c are implanted on inferior somatic nerve $S_l$, unilaterally or bilaterally depending on the response obtained from pre- operative evaluation of responses to stimulation recorded urodynamically.

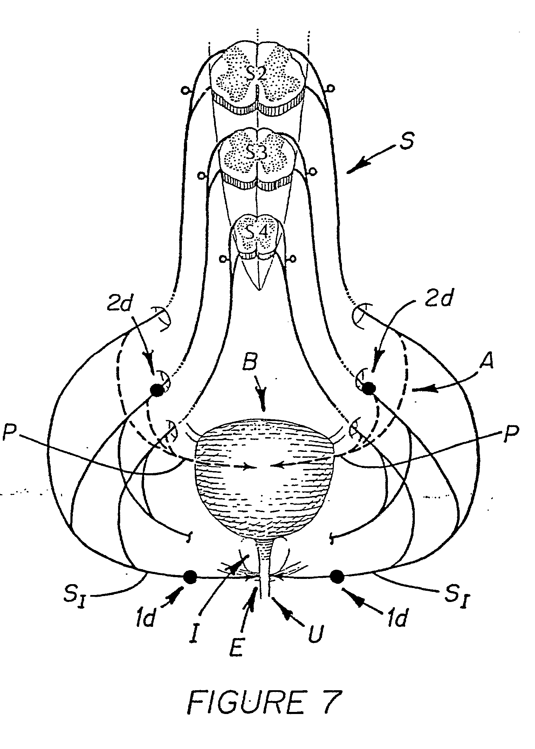

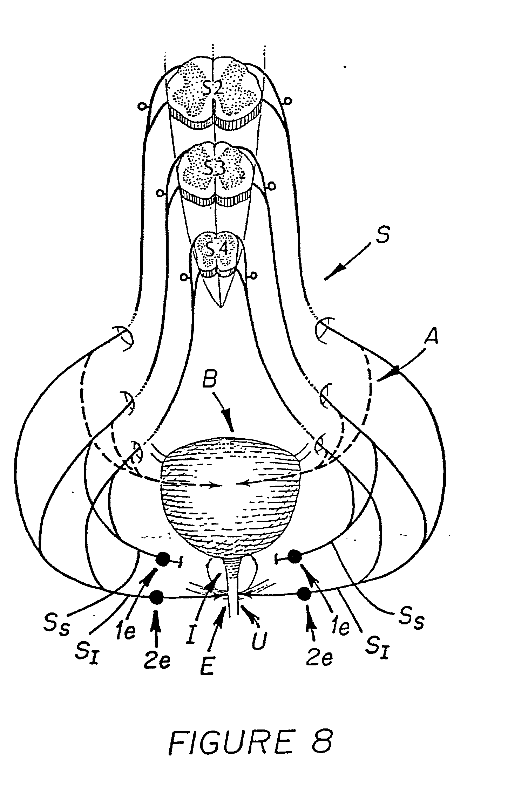

In Figure 7, electrodes 1d are implanted bilaterally on inferior somatic nerve $S_l$ and electrodes 2d are implanted bilaterally on the S3 sacral nerve percutaneously. In Figure 8, electrodes 1e are implanted bilaterally on superior somatic nerve $S_s$ and electrodes 2e are implanted bilaterally on inferior somatic nerve $S_l$.

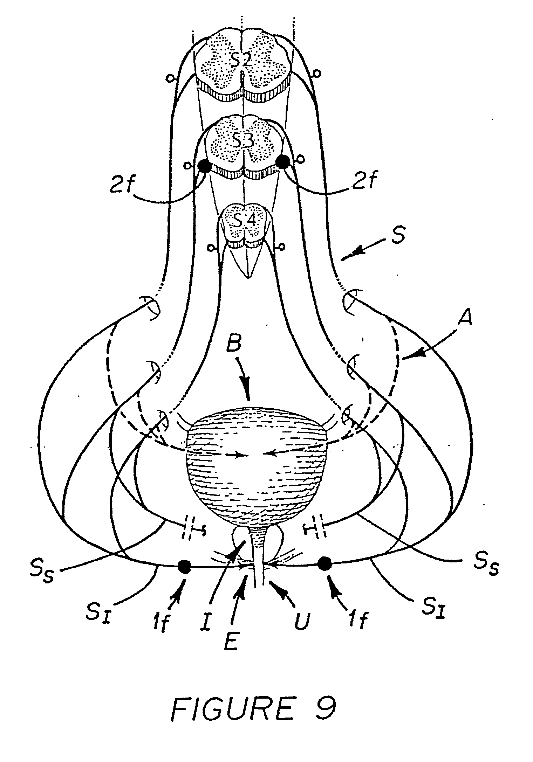

Figure 9 illustrates another operative procedure for controlling continence and bladder contraction. In the illustrated operative procedure, electrodes 1f are implanted bilaterally on inferior somatic nerve $S_l$. Superior somatic $S_s$ is sectioned bilaterally, as illustrated, and a pair of second electrodes 2f are implanted bilaterally on the separated ventral root V of the S3 sacral nerve.

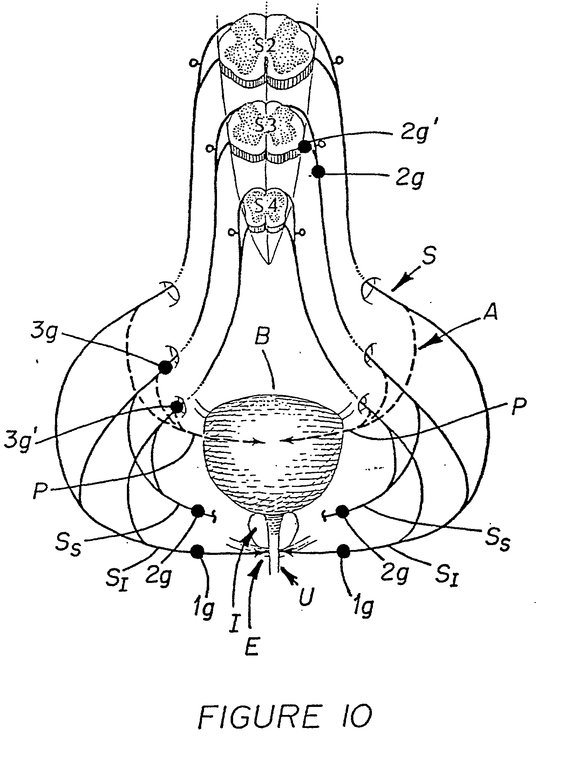

Figure 10 illustrates an operative procedure particularly adapted for achieving continence due to muscle weakness of the bladder or bowel. Electrodes 1g and 2g are implanted bilaterally on inferior somatic nerve $S_l$ and on the S3 sacral nerve, as illustrated. As an option to implantation of the electrode unilaterally on the S3 sacral nerve, an electrode 2g′ could be implanted on the ventral root thereof. In addition, an electrode 3g is implanted unilaterally on the S3 sacral nerve percutaneously. Alternatively, an electrode 3g′ could be implanted on the S4 sacral nerve, also percutaneously.

As another option, illustrated in Figure 10, another electrode 2g could be implanted on superior somatic nerve $S_s$, either unilaterally or bilaterally, as illustrated. The Figure 10 operative procedure illustrates the two components of sphincter contraction with the number of implants and their locations being dependent on recruitability of muscle activity in individual patients and/or the ability of percutaneous technique to adequately couple the electrodes with the appropriate nerve bundles.

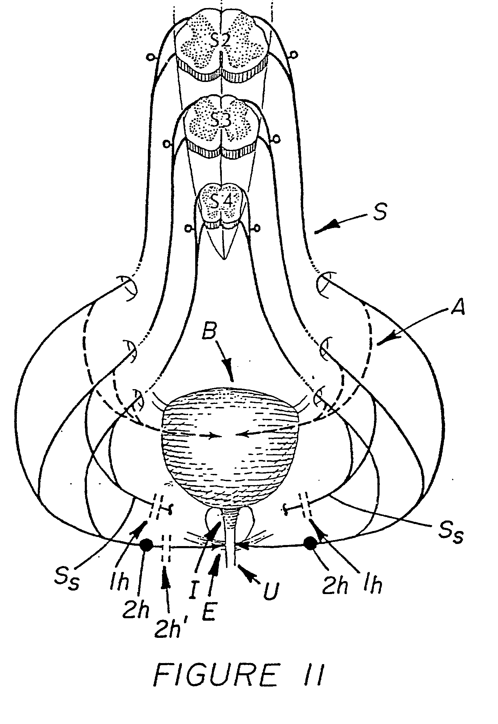

Figure 11 illustrates an operative procedure particularly adapted for controlling autonomic dysreflexia and bladder storage. Superior somatic nerve $S_s$ is sectioned bilaterally at 1h and electrodes 2h are implanted on the inferior somatic nerve bilaterally. Alternatively to the latter step, electrode 2h could be implanted unilaterally with the opposite side of the inferior somatic nerve being sectioned at 2h′.

As indicated above, the operative procedures illustrated in Figures 1 and 3-11 are suggestive of specific procedures applicable to particular patients. Thus, the various steps described above in connection with one particular procedure could be included with or substituted in lieu of steps included in one or more of the other procedures to meet a particular patient's needs. For example, many of the above steps could be performed bilaterally, although disclosed unilaterally, and vice versa.

It follows that the claims appended hereto, when reciting the method steps of "implanting" or "attaching" an electrode to a particular nerve or "sectioning" a particular nerve, etc., intend to cover both unilateral and bilateral procedures.

Selection of the various options described above would be based upon evaluation of responses obtained from pre-operative stimulation recorded urodynamically. The ability of a particular procedure to be conducted percutaneously or surgically, or a combination thereof, further expands application of this invention. Those skilled in the medical arts relating hereto will also appreciate that the above operative procedures may be utilized to control not only bladder functions but also concurrently central functions of other organs, such as the colon, bowel, anal sphincter, etc.

EP 0 245 547 B1

## DESCRIPTION OF MICTURITION CONTROL SYSTEM (FIGS. 12-14)

Figure 12 illustrates a micturition control system adapted to transmit electrical current (radio frequency) pulses to electrodes implanted on selected nerves of the above-described systems, such as the implantations illustrated in Figure 9, i.e., leads 26 and 27 connected to electrodes 1f and leads 47 and 48 connected to electrodes 2f. The control system comprises an external control-transmitter system 10, and a receiver system 11 implanted on a patient for transmitting electrical current pulses to the electrodes. The two-fold purpose of this system is to efficiently (1) maintain urethral tone and hence continence of urine until bladder voiding is desired; and (2) provide bladder contraction and voiding on demand by a patient or his attendant, for patients having bladder or pelvic problems or paralysis.

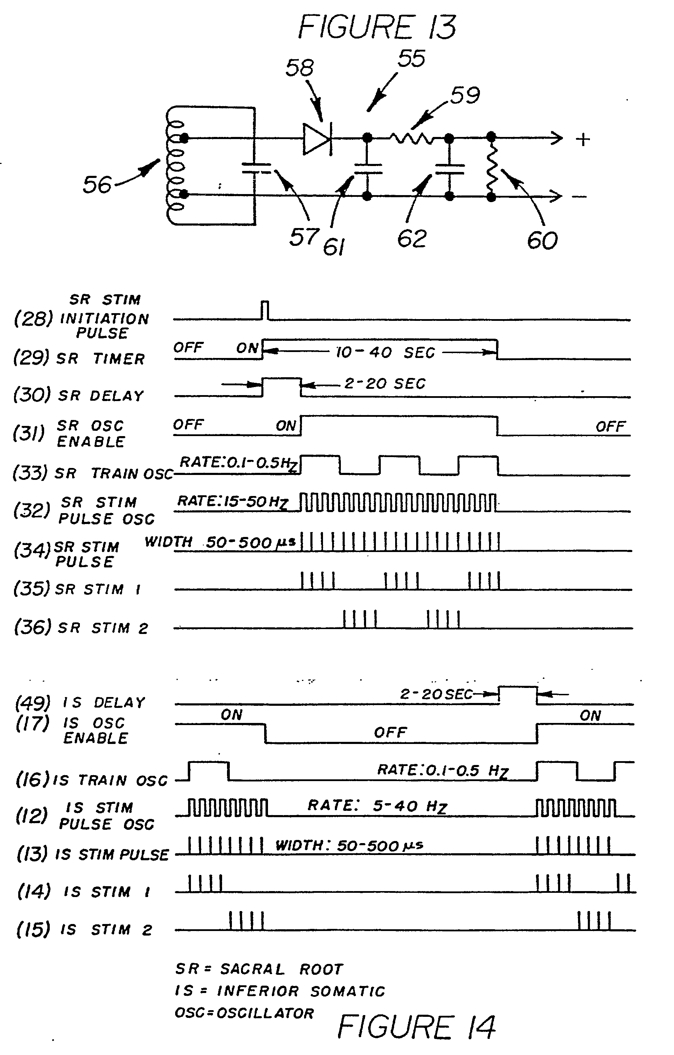

Figure 12 illustrates the electronic signal, transmitter and receiver components of the system whereas Figure 14 illustrates the types of signals and their timed relationship in the electronic control component of the system. The symbol SR as used herein depicts a component of the control system connected to an electrode implanted on a particular sacral nerve or root, whereas IS depicts connection to inferior somatic nerve S$_I$, controlling continence.

The ongoing control of continence (retaining the contents of bladder B) is provided by stimulation of a selected nerve or nerves as described above. This control function is accomplished by the ongoing stimulation produced by a stimulus pulse oscillator 12 (OSC) and associated circuits. The oscillator, preferably operating at a rate within the range of from 5 to 40 pulses per second, emits a square wave output signal that drives and IS stimulus pulse width one-shot (O.S.) 13 which produces signal pulses with widths within the range of from 50 to 500 microseconds, as illustrated in Figure 14. These pulses are controlled by AND gates 14 and 15 to produce separate trains of pulses, also shown in Figure 14. Gating is produced by an IS pulse train duration oscillator 16. It should be noted in Figure 12 that the two phases of the square wave output of oscillator 16 can be obtained by the illustrated inverter circuit.

The frequency of the square wave output of oscillator 16 is preferably selected from within the range of from 0.1 to 0.5 Hertz (cycles per second). This oscillator is controlled by an IS stimulus control flip-flop 17. When the flip-flop is set, it allows oscillator 16 to run to enable gates 14 and 15 to transmit signal pulses (Figure 14) to turn on and off radio frequency (RF) generators or oscillators 18 and 19. Radio frequency amplifiers 20 and 21, whose output amplitudes are adjustable by variable resistors 20' and 21', respectively, receive the separate pulses to drive antennas 22 and 23.

The antennas are inductively coupled to receivers 24 and 25 which detect the separate sets of RF pulses and transmit such detected pulses to a particular nerve-implanted electrodes via electrical leads 26 and 27, respectively. Receivers 24 and 25, as well as hereinafter described receivers 45 and 46, are each subcutaneously implanted on a particular patient.

When it is desired to evacuate bladder B, and assuming that the four energized electrodes are properly implanted for a particular patient in the manner described above, the patient or his attendant will momentarily close a switch 28. Transmitter 10 and attendant antennas 22 and 23 are, of course, suitably housed as an external unit, readily accessible to the patient. The closing of switch 28 will activate an SR stimulus timer 29 for a selected length of time, preferably within the range of from 10 to 40 seconds.

During this time interval, the output Q of timer 29 goes high to activate an SR delay oscillator 30 and resets IS stimulus control flip-flop 17 so that it turns off the IS pulse train duration oscillator 16. As a consequence, stimulation of the urethral sphincter closure, for example, is disabled. Simultaneously therewith, the Q output of timer 29 goes low to allow flip-flop 31 to be set on a signal from SR delay 30. After a predetermined and selected time delay of from 2 to 20 seconds (Figure 14), for example, the output signal from SR delay 30 sets a flip-flop which then starts an SR stimulus pulse oscillator 32 and SR pulse train duration oscillator 33.

Oscillator 32 will generate a square wave signal that is preferably selected from the range of from 15 to 50 Hertz, as diagrammatically illustrated in Figure 14. The output from oscillator 15 then drives an SR stimulus pulse width O.S. 34 which produces signal pulses at a selected width of from 50 to 500 microseconds. These pulses are ANDED by gates 35 and 36 with the output of oscillator 33 to produce trains of pulses, as illustrated in Figure 14. It should be noted in Figure 12 that the opposite phases of the square wave output of oscillator 33 may also be obtained by the use of the illustrated inverter circuit. Gate control originates at oscillator 33 which has a selected frequency within the range of from 0.1 to 0.5 Hertz.

The pulse trains from AND gates 35 and 36 control RF oscillators 37 and 38, respectively. These are turned on when the pulses are high. The RF signals are amplified in RF amplifiers 39 and 40 wherein the output amplitudes are controlled by variable resistors 39' and 40', respectively. The outputs of the amplifiers drive antennas 43 and 44 which inductively couple the signal through the patient's skin and to implanted receivers 45 and 46. The receivers detect the RF pulses and transmit the stimulus pulses to the particular implanted electrodes, via electrical leads 47 and 48.

At the end of the selected time period, e.g., 10 to 40 seconds, of timer 29, the output reverses, as illustrated in Figure 14. The Q output goes low and enables flip-flop 17 to activate oscillator 16 when the "set" signal is transmitted from IS delay O.S. 49. The Q output of timer 29 goes high and activates 49. At the end of the preselected time delay of from 2 to 20 seconds, the output (Figure 14) sets IS stimulus circuitry until bladder evacuation is again required. The Q output from timer 29 also resets flip-flop 31 and

7

thus disables oscillators 32 and 33 to end the bladder voiding stimulation.

The above oscillators (OSC) may be made of the astable multivibrator type, manufactured by Intersil Corp., U.S.A., under Model No. 1CM7556. The one-shot (O.S.) monstable multivibrators may also be of the common type manufactured by Intersil Corp. The flip-flops (F.F.) may constitute the type manufactured by RCA Corporation, U.S.A., under Model No. CD4027B, whereas the gates may be of the type manufactured by the same company under Model No. DC4081B.

With regards to the micturition control system just described, the receivers 24 and 25, leads 26 and 27 and electrodes 1f comprise a first stimulation system for stimulating the external sphincter functions while receivers 45 and 46, leads 47 and 48 and electrodes 2f comprise a second stimulation system for stimulating the bladder functions. Elements 12–16 and 18–23 comprise a first pulse generating and transmitting means for generating and transmitting a series of electrical pulses to the receivers 24 and 25 of the first stimulation system, while elements 32–40, 43 and 44 comprise a second pulse generating and transmitting means for generating and transmitting series of pulses to the receivers 45 and 46 of the second stimulation system.

Receivers 24, 25, 45 and 46 may constitute a standard implantable silastic-coated unit containing an antenna coil adapted to receive the "rf" pulses transmitted from their respective transmitter antennas 22, 23, 43 and 44. For example, each receiver may be similar to the type manufactured by Avery Laboratories, Inc., U.S.A., under Model No. I-110 (bipolar). Figure 13 illustrates a typical circuit 55 for the receiver.

In particular, a coil 56 functions as an antenna that receives the inductive signal transmitted thereto by a respective transmitter antenna which is placed externally of the patient, adjacent to the location of the implanted receivers. A capacitor 57, in conjunction with coil 56, provides a tuned circuit that is tuned to one of the four different frequencies of the transmitter. The other three receivers are tuned to their respective transmitting frequencies when the system uses four separate radio frequencies. Alternatively, the frequencies may be the same for all four transmitters with the four receivers being tuned to the same transmitting frequency. In the latter application, the four transmitting antennas and the four implanted receivers must be separated so that the signal in any one transmitting antenna will not provide false signals in the other three receivers.

Still referring to Figure 13, a diode 58 detects, by half-wave rectification, the pulsed stimulus current from the RF bursts. Resistors 59 and 60 and capacitors 61 and 62 function to filter the RF out of the stimulus signal which is lead to the nerve electrodes via electrical leads, described above. Maximum stimulation of the nerves is achieved when the negative pole is attached to the distal electrode contact on the nerve and the corresponding positive pole is the proximal contact of the nerve electrode assembly.

Those skilled in the art will appreciate that the Figure 12 control system can be suitably modified to control the energization of the electrodes used in the above-described procedures and variations thereof. For example, only the portion of the system for controlling pulse imputs to antennas 24 and 25 could be utilized to energize electrodes 2 in Figure 1.

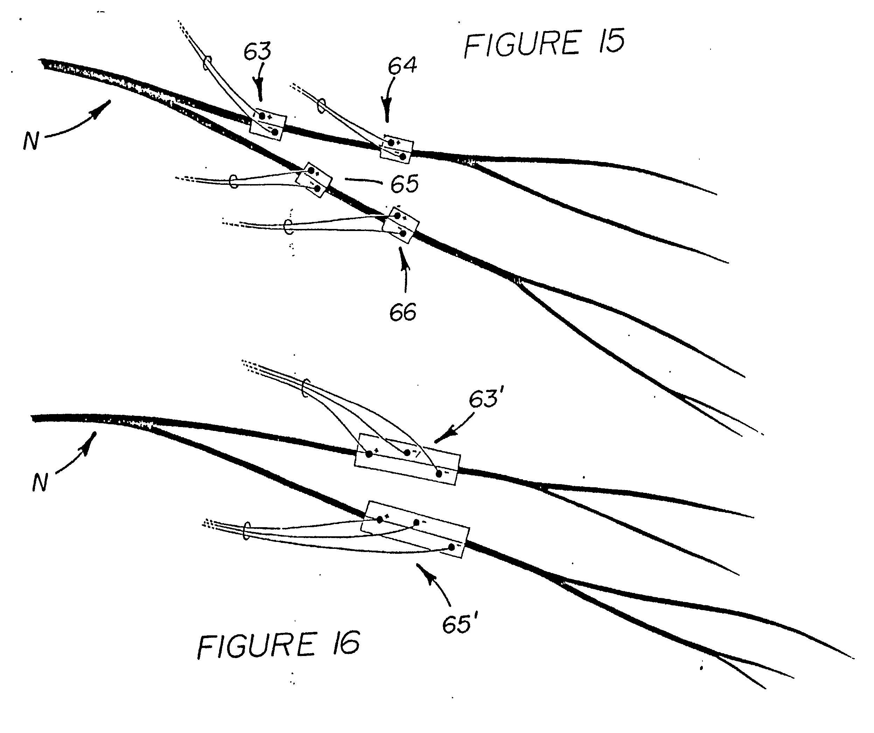

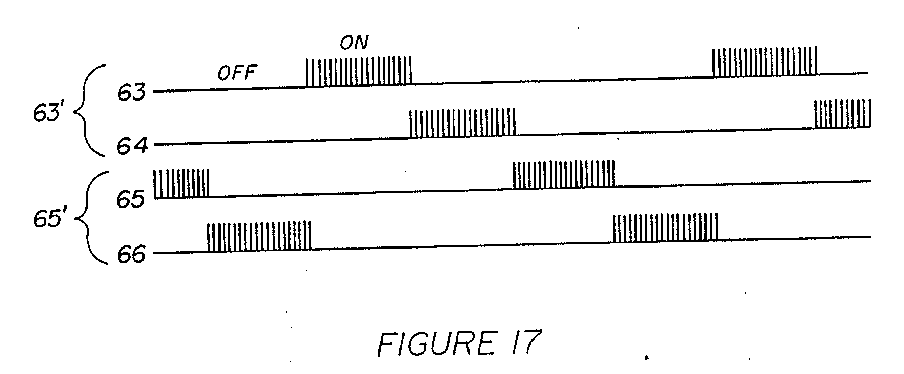

Figures 15 and 16 illustrate typical electrode implantations for stimulation purposes. Figure 17 diagramatically illustrates the timing of stimulus pulse trains to electrode pairs with each electrode contact being activated essentially 100/n % of the time for n active (cathodol) contacts.

One of the primary problems encountered with prolonged and continuous electrical stimulation of a nerve and muscle system to achieve chronic on-going muscle contractions, is fatigue of the nerve and associated muscles. One way to prevent or prolong the onset of fatigue (the muscle being more susceptible to fatigue than the nerve) is to stimulate the system in a non-continuous and time-modulated format. Otherwise stated, alternate stimulation of different groups of nerve bundles in a nerve system with short bursts of stimulus pulses provides such desiderata. This method of stimulation allows the muscles and nerves to recover between trains of stimuli while other nerves and muscles are being activated to continue the desired physiological effect.

Time-modulation of stimuli to nerves to achieve muscle contraction, for example, can be accomplished in at least two ways. Figure 15 illustrates a first approach wherein a multiplicity of electrode pairs 63, 64 and 65, 66 are attached to separate nerve bundles. Each pair of electrodes are activated so that each electrode is "on" for only a portion of a particular stimulation cycle, e.g., with four electrode pairs, each would be stimulating its nerve bundle approximately one-quarter of the time (in general, 100/n % of the time for n electrode pairs). It should be noted in Figure 7, although shown in coincidence, that an overlap or dead time can be affected between the stimuli trains.

Figure 16 illustrates a second approach to accomplish time modulation of nerve stimulation wherein a multiplicity of active electrode contacts are employed on a single electrode. However, in order to achieve this alternating stimulation on a single nerve bundle, the intensity of the stimulus pulses must be sufficiently low so that all nerve bundles are not stimulated by a single active electrode contact. Nonetheless, the amplitude of the stimulus pulses must be sufficiently high so that the desired physiological function can be achieved.

The active electrodes (cathodes) must be located on the nerve bundle so that they stimulate different nerve bundles. Thus, a single electrode is not activating all of the nerve bundles in associate muscles, as illustrated in Figures 16 and 17. Ideally, each electrode contact would function to activate the proper proportional number of fibres, e.g., in the case of two contacts each contact would activate one-half of the nerve bundles and in the case of four contacts each contact would active one-quarter of the nerve bundles, etc. However, the desired physiological function must be achieved when a single contact is used on that particular nerve bundle. An exception, of course, would be in applications wherein other nerve bundles are similarly connected to a similar time-modulated system. In the latter application, partial nerve stimulation from the combined group of stimulated nerve bundles would accomplish the desired physiological results.

## ELECTRICAL CONTROL OF VISCERAL, VISCERO-SOMATIC, AND SOMATIC NEUROMUSCULAR DYSFUNCTIONS

The physiology of the bladder and bowel is closely linked to the urethral muscle physiology of the pelvic floor and its related urethral and anal sphincters. The sequences for storage (continence) and evacuation suggest that the somatic muscles of the pelvic floor are principally responsible for both continence and evacuation. During the storage phase, the visceral organs, i.e., bowel/bladder, are either released from the reflex inhibition or are directly facilitated into contracting, or both. It has been determined that neural control of the pelvic muscles largely determines the state of activity of the pelvic viscera (bowel, bladder, and possible erection). A simple example is the "hold" reflex used to suppress a strong urge to void or defecate at inconvenient times.

If the neural regulation of bladder and bowel activity is directly tied to that of the pelvic muscles in the normal, then it is most certainly tied to it in the abnormal. Just as the hold reflex is used to suppress an inconvenient urge to empty either the bladder or bowel, an electrically induced contraction of the pelvic sphincters can be used to suppress an overly active bladder or bowel.

There is a broad spectrum of patients who experience a multitude of symptoms resulting from dysfunctional behavior of the bladder, bowel, urethra, anal and pelvic floor muscle systems. Not uncommonly, the muscle dysfunction cannot be ascribed to any disease. The muscle dysfunction can, however, be very similar to that foud for other neural disorders (e.g., meningomyelocele, hydrocephalus, spinal injury, multiple scerosis, stroke, etc.). The visceral dysfunction can be demonstrated especially in the case of the bladder--to be associated with pelvic muscle dysfunction, with the behavior of the bladder being a direct result of excessive inhibition (e.g., inability to void completely because of an inability to relax the urinary sphincter completely), or excessive triggering of bladder contractions (i.e., a precipitate urge to void one's bladder) because of a breakdown in the efficiency of reflex coordination between the bladder and pelvic muscles. A similar analogy can be made for problems affecting the bowel (as well as erection). Correction of the pelvic muscle dysfunction can thus serve to correct the visceral muscle dysfunction. Other effects described by patients have included the reduction of severe neck spasm, back spasm and leg cramps.

Visceral muscle dysfunctions which can be considered a result of overfacilitated activity include the spastic colon, interstitial cystitis, detrusor instability, cardiovascular problems, such as migraine headache or palpatations, and bladder retention syndromes. Somatic muscle dysfunctions directly resulting from poor neural regulation and overfacilitated behavior include: pelvic pain syndromes, frequency syndromes (pelvic floor and/or urethral instability), incontinency due to poor relaxation or instability of the sphincters (either bowel or bladder), and incontinence following prostatectomy.

Each of the above has been shown to respond to stimulation of the somatic muscles of the pelvic floor (primarily levator ani muscle I in Figure 18). Neurostimulation of the pelvic muscles has a stabilizing effect on their neuro-regulatory mechanisms. Behavioral stabilization of pelvic muscles then affects the neuro control of the viscera.

Because of the similarity in nervous control between the bowel and bladder, the following bowel problems may also be treatable by a sacral or pudendal nerve electrode implant, namely, "spastic colon", and fecal incontinence either from spasticity or incompetence of the anal sphincter, and infrequent or too frequent bowel movements.

A spinoff benefit that has been noted is the treatment of foot drop. It appears that the planter flexion of the distal half of the foot and toes gives added stability to the gait. It has long been believed that foot drop was the result of a weakness in the muscles controlled by the perineal nerve. Stimulation of this nerve has been used to lift the foot using the foot dorsiflexors, but with limited success. Foot drop has been shown to improve by stimulation of sacral S3 nerve root N3 because of a better push the foot has as a result of planter flexion.

Figure 18 illustrates letters depicting various components of the pelvic plexus region of a human that are common to those shown in Figures 1-11. The following listing includes newly discussed components, shown in Figure 18, as well as the common ones:

A: Autonomic nerve system.
B: Bladder.
C1-C6: Electrodes, shown as cuff electrodes for illustration purposes (other types could be used).
D: Dorsal root of nerve (sensory).
E: External sphincter of bladder B.
F: Foramen electrode.

I: Internal sphincter or levator ani muscle (pelvis floor, i.e., the composite muscular structure that constitutes the outlet of the boney pelvis and primarily consisting of the levator ani muscle).

J: Dorsal nerve of the penis.

L: Anal branch of pudendal nerve T.

N2, N3: Sacral nerves originating at sacral segments S2 and S3, respectively.

P: Pelvic nerves connected between the sacral nerves and the detrusor muscle of bladder B.

R: Anal sphincter (sphincter ani).

$S_I$: Inferior somatic nerve.

$S_S$: Superior somatic nerve.

T: Pudendal nerve.

U: Urethra.

V: Ventral root of nerve (motor).

Methods herein disclosed can be used to either modulate symptoms resulting from a loss of coordination between the normally synchronized functions of organs, including bladder B, rectum R and associated bladder sphincters E and I and the anal sphincter for rectum R (Chart I), or to treat incontinence by increasing sphincter tonus (Chart II). Sacral nerves N2 and N3 originate at sacral segments S2 and S3, respectively, and form pelvic nerve P that controls contraction of a detrusor muscle surrounding bladder B. The sacral nerves also form somatic components that subdivide into: (1) superior somatic nerve $S_S$; and (2) pudendal nerve T that includes (a) inferior somatic nerve $S_I$ connected to muscles controlling external sphincter E of bladder B, (b) anal branch L connected to the anal sphincter for rectum R, and (c) dorsal nerve J connected to the penis. The nerve bundles connected to the various sphincters are controllable at a lower level of electrical stimulation that that required to control the muscles for the bladder and rectum proper.

Figure 18 illustrates six cuff electrodes C1-C6 adapted to be positioned on selected nerve bundles (while simultaneously isolating adjacent nerve bundles) either individually or in combination with at least one other electrode for stimulation purposes. Such positioning step occurs after identifying the anatomical location and functional characteristics of the selected nerve bundle or bundles. Pulse trains are then applied sequentially to the electrode or electrodes to control the function of the organ.

Individually, electrodes C1-C6 modulate or control the function(s) of the following organs:

C1: Bladder B, sphincter E, anal sphincter R and the detrusor muscle for bladder B.

C2: The detrusor muscle for bladder B and both bladder and anal sphincters E and R

C3: Both bladder and anal sphincters E and R.

C4: Internal sphincter 1 (pelvic floor).

C5: Bladder sphincter E.

C6: Anal sphincter R.

CHART I

The following chart indicates twenty-one different combinations of electrode placement (unilaterally or bilaterally) for modulating the above-discussed symptoms resulting from a loss or coordination between a persons's organs, including bladder B, rectum R and associated sphincters:

| Organ(s) Affected | C1 Sacral Nerve N2 | C2 Sacral Nerve N3 | C3 Pudendal Nerve T | C4 Superior Somatic Nerves $^S$S | C5 Inferior Somatic Nerve $^S$I | C6 Anal Branch L |
|---|---|---|---|---|---|---|
| (1) B, E, R | X | | X | | | |
| (2) B, E, R, I | X | | | X | | |
| (3) B, E, R | X | | | | X | |
| (4) B, E, R | X | | | | | X |
| (5) B, E, R, I | X | | X | X | | |
| (6) B, E, R, I | X | | | X | X | |
| (7) B, E, R, I | X | | | X | | X |
| (8) B, E, R | | X | X | | | |
| (9) B, E, R, I | | X | | X | | |
| (10) B, E, R | | X | | | X | |
| (11) B, E, R | | X | | | | X |
| (12) B, E, R, I | | X | X | X | | |
| (13) B, E, R, I | | X | | X | X | |
| (14) B, E, R | | X | | X | | X |
| (15) B, E, R | X | X | X | | | |
| (16) B, E, R, I | X | X | | X | | |
| (17) B, E, R | X | X | | | X | |
| (18) B, E, R | X | X | | | | X |
| (19) B, E, R, I | X | X | X | X | | |
| (20) B, E, R, I | X | X | | X | | |
| (21) B, E, R, I | X | X | | X | X | |

## CHART II

The following second chart indicates electrode placement (unilaterally or bilaterally) for treatment of incontinence by increasing sphincter tonus either by direct stimulation of a sphincter muscle or by modulating reflex control mechanisms so that more effective sphincter tonus results:

| Organ(s) Affected | C3 Pudendal Nerve T | C4 Superior Somatic Nerves $^S$S | C5 Inferior Somatic Nerve $^S$I | C6 Anal Branch |
|---|---|---|---|---|
| (1) E, R | X | | | |
| (2) I (Pelvic Floor) | | X | | |
| (3) E | | | X | |
| (4) R | | | | X |
| (5) E, R, I | X | X | | |
| (6) I, E | | X | X | |
| (7) I, R | | X | | X |

The term "reflex control mechanisms" means those nerve bundles that control interrelated activity between bladder B and pelvic floor musculature (primarily levator ani muscle I) as they can reflexively influence each other by either inhibition or facilitation.

11

It should be noted in the charts that various electrode combinations may affect the identical organs, but to different degrees of intensity. For example, although electrode combinations (1) and (8) in Chart I each affect bladder B, bladder sphincter E and anal sphincter R, in combination (8) the bladder will be relatively more responsive since the main pelvic nerve supply P emanates primarily from sacral segment S3 and to a lesser amount from sacral segment S2.

The site or sites chosen for implantation of an electrode is determined by careful evaluation of a patient's problems. Such evaluations consist of symptom analysis, physical deficits or variations in muscle behavior of the lower extremities and pelvic muscles, or loss of sensation, the results of urodynamic testing and the results of test stimulation of the various sacral nerves. A temporary electrode is normally inserted percutaneously into one or more of the sacral foramena and specific nerve roots test stimulated for a response. When a desired response is obtained, a temporary electrode can be "floated" (e.g., Foramene electrode F in Figure 18) in the vicinity of the nerve or nerves. This procedure allows the patient to have a three to five day trial of stimulation to evaluate the therapeutic benefits of stimulation.

Based on the response obtained with the test stimulation, the patient can be further evaluated for the response to be obtained by percutaneously implanting an electrode on one or more of the selected nerve bundles or an electrode can be permanently implanted, either via sacral laminectomy and placement of an electrode directly on a specific sacral nerve or by placing an electrode on the sacral foramene without performing a laminectomy. Therapeutic benefits are thus obtained by stimulation of specific pelvic muscles.

The electronic control system and electrode arrangements shown in Figures 12-17 can also be used to effect the operative procedures set forth in Charts I and II, with appropriate modifications for each specific procedure.

## Claims

1. Apparatus for controlling the functions of a bladder or bowel and its associated external sphincter in an anatomical system of a selected human, comprising:

a first stimulation system (24–27, 1f) for implantation in said human and including at least one receiver (24) and an electrode (1f) associated therewith, the electrode being positionable on a nerve controlling the function of said external sphincter,

a second stimulation system (45–48, 2f) for implantation in said human and including at least one receiver (45) and an electrode (2f) associated therewith, the electrode being positionable on a nerve controlling the function of said bladder,

a first pulse generating and transmitting means (12–16, 18–23), normally being in on a state, for generating and transmitting a series of electrical pulses to the receiver (24) of said first stimulation system,

a second pulse generating and transmitting means (32–40, 43, 44), normally being in an off state, for generating and transmitting a series of electrical-pulses to the receiver (45) of said second stimulation system, and

a manually operable switch (28), means (29, 17) for turning off said first pulse generating and transmitting means in response to operation of said manually operable switch, and, after a first preselected time delay (30), for turning on said second pulse generating and transmitting means,

means (29, 31) operable at a selected time period after turning on of said second pulse generating and transmitting means for turning off said second pulse generating and transmitting means and, after a second preselected time delay (49), for turning back on said first pulse generating and transmitting means.

2. Apparatus as claimed in claim 1, characterised by a timer (29) having a selected time period, and wherein said means (29, 17) for turning off said pulse generating and transmitting means in response to operation of said manually operable member also starts said timer (29) in response to operation of said manually operable member (28) and wherein said means (29, 31) for turning off said second pulse generating and transmitting means is the selected time period of said timer (29).

3. Apparatus as claimed in claim 1, characterised in that each of said first (12–16, 18–23) and second (32–40, 43, 44) pulse generating and transmitting means has the function of generating and transmitting time spaced bursts of pulses.

4. Apparatus as claimed in claim 1, characterised in that said first stimulation system (24–27, 1f) includes a second receiver (25) and an electrode (1f) associated with said second receiver, the two electrodes of said first stimulation system being positionable on nerves both controlling the function of said external sphincter, and wherein said first pulse generating and transmitting means (12–16, 18–23) has the function of generating and transmitting a series of electrical pulses to the two receivers (24, 25) of said first stimulation system.

5. Apparatus as claimed in claim 4 characterised in that said first pulse generating and transmitting means (12–16, 18–23) has the function of generating and transmitting time spaced bursts of pulses separately to each of the two receivers (24, 25) of the first stimulation system.

6. Apparatus as claimed in claim 5, characterised in that said first pulse generating and transmitting means (12–16, 18–23) has the function of generating and transmitting bursts of pulses alternately to the two receivers (24, 25) of the first stimulation system.

7. Apparatus claimed in claim 1, characterised in that said second stimulation system (45–48, 2f) includes a second receiver (46) and an electrode 2f associated with said second receiver, the two electrodes of said second stimulation system being positionable on nerves both controlling the function of said bladder or bowel associated with said external sphincter, and wherein said second pulse generating and transmitting means (32–40, 43, 44) has the function of generating and transmitting a series of electrical pulses to the two receivers (45, 46) of said second stimulation system.

8. Apparatus as claimed in claim 7, characterised in that said second pulse generating and transmitting means (32–40, 43, 44) has the function of generating and transmitting time spaced bursts of pulses separately to each of the two receivers (45, 46) of the second stimulation system.

9. Apparatus as claimed in claim 8, characterised in that said second pulse generating and transmitting means (32–40, 43, 44) has the function of generating and transmitting the burst of pulses alternately to the two receivers (45, 46) of the second stimulation system.

10. Apparatus as claimed in claim 1, characterised in that said first stimulation system (24–27, 1f) includes a second receiver (25) and an electrode (1f) associated therewith, the two electrodes of said first stimulation system being positionable on nerves both controlling the function of said external sphincter, wherein said second stimulation system (45–48, 2f) includes a second receiver (46) and an electrode (2f) associated therewith, the two electrodes (2f) of said second stimulation system being positionable on nerves both controlling the function of said bladder or bowel associated with said external sphincter, wherein said first pulse generating and transmitting means (12–16, 18–23) has the function of generating and transmitting a series of electrical pulses to the two receivers (24, 25) of said first stimulation system, and wherein said second pulse generating and transmitting means (32–42,43,44) has the function of generating and transmitting a series of electrical pulses to the two receivers (45, 46) of said second stimulation system.

11. Apparatus as claimed in claim 10, characterised in that said first pulse generating and transmitting means (12–16, 18–23) has the function of generating and transmitting time spaced bursts of pulses separately to each of the two receivers (24, 25) of the first stimulation systems, and wherein said second pulse generating and transmitting means (32–40, 43, 44) has the function of generating and transmitting time spaced burst of pulses separately to each of the two receivers (45, 46) of the first stimulation system, and wherein said second pulse generating and transmitting means (32–40, 43, 44) has the function of generating and transmitting time spaced bursts of pulses separately to each of the two receivers (45, 46) of the second stimulation system.

12. Apparatus as claimed in claim 11 characterised in that said first pulse generating and transmitting means (12–16, 18–23) has the function of generating and transmitting the bursts of pulses alternately to the two receivers (45, 46) of the first stimulation system, and wherein said second pulse generating and transmitting means (32–40, 43, 44) has the function of generating and transmitting the bursts of pulses alternately to the two receivers (45, 46) of the second stimulation system.

## Patentansprüche

1. Vorrichtung zum Steuern der Funktion einer Blase oder eines Darmes und des jeweiligen äußeren Schließmuskels im anatomischen System eines bestimmten Menschen, gekennzeichnet durch:

ein erstes in den Menschen zu implantierendes Stimulierungssystem (24–27, 1f), das mindestens einen Empfänger (24) und eine damit verbundene Elektrode (1f) enthält, wobei die Elektrode an einen Nerv anschließbar ist, der die Funktion des äußeren Schließmuskels steuert,

ein zweites in den Menschen zu implantierendes Stimulierungssystem (45–48, 2f), das mindestens einen Empfänger (45) und eine damit verbundene Elektrode (2f) enthält, wobei die Elektrode an einen Nerv anschließbar ist, der die Funktion der Blase steuert,

ein erstes impulserzeugendes und -übertragendes Mittel (12–16, 18–23), das normalerweise "Ein"-geschaltet ist, zum Erzeugen und Übertragen einer Reihe von elektrischen Impulsen zum Empfänger (24) des ersten Stimulierungssystems,

ein zweites impulserzeugendes und -übertragendes Mittel (32–40, 43, 44), das normalerweise "Aus"-geschaltet ist, zum Erzeugen und Übertragen einer Reihe von elektrischen Impulsen zum Empfänger (45) des zweiten Stimulierungssystems,

einen per Hand betätigbaren Schalter (28),

ein Mittel (29, 17) zum Ausschalten des ersten impulserzeugenden und -übertragenden Mittels als Reaktion auf die Betätigung des per Hand betätigbaren Schalters, und, nach einer ersten vorgewählten Zeitverzögerung (30), zum Einschalten des zweiten impulserzeugenden und -übertragenden Mittels,

ein Mittel (29, 31), das nach einer gewählten Zeitspanne nach Einschalten des zweiten impulserzeugenden und -übertragenden Mittels betriebsbereit ist, zum Ausschalten des zweiten impulserzeugenden und -übertragenden Mittels und zum Wiedereinschalten des ersten impulserzeugenden und -übertragenden Mittels nach einer zweiten vorgewählten Zeitverzögerung (49).

2. Vorrichtung nach Anspruch 1, dadurch gekennzeichnet, daß sie einen Zeitgeber (29) mit einer vorwählbaren Zeitspanne aufweist, wobei das Mittel (29, 17) zum Ausschalten des impulserzeugenden und -übertragenden Mittels als Reaktion auf die Betätigung des per Hand betätigbaren Schalters auch diesen Zeitgeber (29) als Reaktion auf die Betätigung des per Hand betätigbaren Schalters (28) einschaltet, und wobei das Mittel (29, 31) zum Ausschalten des zweiten impulserzeugenden und -übertragenden Mittels die gewählte Zeitspanne des Zeitgebers (29) ist.

3. Vorrichtung nach Anspruch 1, dadurch gekennzeichnet, daß jedes erste (12–16, 18–23) als auch zweite (32–40, 43, 44) impulserzeugende und -übertragende Mittel die Funktion des Erzeugens und Übertragens von zeitunterteilten Impulsstößen hat.

4. Vorrichtung nach Anspruch 1, dadurch gekennzeichnet, daß das erste Stimulierungssystem (24–27, 1f) einen zweiten Empfänger (25) und eine mit dem zweiten Empfänger verbundene Elektrode (1f) enthält, wobei die beiden Elektroden des ersten Stimulierungssystems an Nerven anschließbar sind, und beide die Funktion des externen Schließmuskels überwachen, und daß das erste impulserzeugende und -übertragende Mittel (12–16, 18–23) die Funktion des Erzeugens und Übertragens einer Reihe von elektrischen Impulsen zu den beiden Empfängern (24, 25) des ersten Stimulierungssystems hat.

5. Vorrichtung nach Anspruch 4, dadurch gekennzeichnet, daß das erste impulserzeugende und -übertragende Mittel (12–16, 18–23) die Funktion des Erzeugens und Übertragens von zeitunterteilten Impulsstößen getrennt zu jedem der beiden Empfänger (24, 25) des ersten Stimulierungssystems hat.

6. Vorrichtung nach Anspruch 5, dadurch gekennzeichnet, daß das erste impulserzeugende und -übertragende Mittel (12–16, 18–23) die Funktion des Erzeugens und des Übertragens von Impulsstößen abwechselnd zu den beiden Empfängern (24, 25) des ersten Stimulierungssystems hat.

7. Vorrichtung nach Anspruch 1, dadurch gekennzeichnet, daß das zweite Stimulierungssystem (45–48, 2f) einen zweiten Empfänger (46) und eine mit dem zweiten Empfänger verbundene Elektrode (2f) enthält, wobei die beiden Elektroden des zweiten Stimulierungssystems an Nerven anschließbar sind und beide die Funktion der Blase oder des Darms mit dem zugeordneten äußeren Schließmuskel steuern, und daß das zweite impulserzeugende und -übertragende Mittel (32–40, 43, 44) die Funktion des Erzeugens und Übertragens einer Reihe von elektrischen Impulsen zu den beiden Empfängern (45, 46) des zweiten Stimulierungssystems hat.

8. Vorrichtung nach Anspruch 7, dadurch gekennzeichnet, daß das zweite impulserzeugende und -übertragende Mittel (32–40, 43, 44) die Funktion des Erzeugens und Übertragens von zeitunterteilten Impulsstößen getrennt zu jedem der beiden Empfänger (45, 46) des zweiten Stimulierungssystems hat.

9. Vorrichtung nach Anspruch 8, dadurch gekennzeichnet, daß das zweite impulserzeugende und -übertragende Mittel (32–40, 43, 44) die Funktion des Erzeugens und Übertragens von Impulsstößen abwechselnd zu den beiden Empfängern (45, 46) des zweiten Stimulierungssystems hat.

10. Vorrichtung nach Anspruch 1, dadurch gekennzeichnet, daß das erste Stimulierungssystem (24–27, 1f) einen zweiten Empfänger (25) und eine damit verbundene Elektrode (1f) enthält, wobei die beiden Elektroden des ersten Stimulierungssystems an Nerven anschließbar sind und beide die die Funktion des äußeren Schließmuskels steuern, daß das zweite Stimulierungssystem (45–48, 2f) einen zweiten Empfänger (46) und eine damit verbundene Elektrode (2f) enthält, wobei die beiden Elektroden (2f) des zweiten Stimulierungssystems an Nerven anschließbar sind und beide die Funktion der Blase oder des Darmes mit dem zugeordneten äußeren Schließmuskel steuern, daß das erste impulserzeugende und -übertragende Mittel (12–16, 18–23) die Funktion des Erzeugens und Übertragens einer Reihe von elektrischen Impulsen zu den beiden Empfängern (24, 25) des ersten Stimulierungssystems und daß das zweite impulserzeugende und -übertragende Mittel (32–42,43,44) die Funktion des Erzeugens und Übertragens einer Reihe von elektrischen Impulsen zu den beiden Empfängern (45, 46) des zweiten Stimulierungssystems hat.

11. Vorrichtung nach Anspruch 10, dadurch gekennzeichnet, daß das erste impulserzeugende und -übertragende Mittel (12–16, 18–23) die Funktion des Erzeugens und Übertragens von zeitunterteilten Impulsstößen getrennt zu jedem der beiden Empfänger (24, 25) des ersten Stimulierungssystems hat, daß das zweite impulserzeugende und -übertragende Mittel (32–40, 43, 44) die Funktion des Erzeugens und Übertragens von zeitunterteilten Impulsstößen getrennt zu jedem der beiden Empfänger (45, 46) des ersten Stimulierungssystems hat, und daß das zweite impulserzeugende und -übertragende Mittel (32–40, 43, 44) die Funktion des Erzeugens und Übertragens von zeitunterteilten Impulsstößen getrennt zu jedem der beiden Empfänger (45, 46) des zweiten Stimulierungssystems hat.

12. Vorrichtung nach Anspruch 11, dadurch gekennzeichnet, daß das erste impulserzeugende und -übertragende Mittel (12–16, 18–23) die Funktion des Erzeugens und Übertragens der Impulsstöße abwechselnd zu den beiden Empfängern (45, 46) des ersten Stimulierungssystems hat, und daß das zweite impulserzeugende und -übertragende Mittel (32–40, 43, 44) die Funktion des Erzeugens und Übertragens der Impulsstöße abwechselnd zu den beiden Empfängern (45, 46) des zweiten Stimulierungssystems hat.

**Revendications**

1. Appareil de commande des fonctions d'une vessie ou d'intestins et leur sphincter extérieur correspondant dans un système anatomique d'un être humain choisi, comprenant:
un premier système de stimulation (24–27, 1f) pour l'implantation dans ledit être humain et comprenant au moins un récepteur (24) et une électrode (1f) y associée, l'électrode pouvant être positionnée sur un nerf commandant la fonction dudit sphincter extérieur,

un second système de stimulation (45–48, 2f) pour l'implantation dans ledit être humain et comprenant au moins un récepteur (45) et une électrode (2f) y associée, l'électrode pouvant être positionnée sur un nerf commandant la fonction de ladite vessie,

un premier moyen d'engendrement et de transmission d'impulsions (12–16, 18–23), normalement à l'état enclenché, pour l'engendrement et la transmission d'une série d'impulsions électriques au récepteur (24) dudit premier système de stimulation,

un second moyen d'engendrement et de transmission d'impulsions (32–40, 43, 44), normalement à l'état déclenché, pour l'engendrement et la transmission d'une série d'impulsions électriques au récepteur (45) dudit second système de stimulation, et

un interrupteur à commande manuelle (28),

des moyens (29, 17) pour déclencher ledit premier moyen d'engendrement et de transmission d'impulsions en réponse au fonctionnement dudit interrupteur à commande manuelle, et, après une première temporisation présélectionnée (30), pour enclencher ledit second moyen d'engendrement et de transmission d'impulsions,

des moyens (29, 31) pouvant être commandés à un intervalle de temps choisi après l'enclenchement dudit second moyen d'engendrement et de transmission d'impulsions pour le déclenchement dudit second moyen d'engendrement et de transmission d'impulsions et, après une seconde temporisation présélectionnée (49), pour réenclencher ledit premier moyen d'engendrement et de transmission d'impulsions.

2. Appareil tel que revendiqué à la revendication 1, caractérisé en ce qu'un temporisateur (29) ayant une temporisation sélectionnée et dans lequel lesdits moyens (29, 17) pour le déclenchement dudit moyen d'engendrement et de transmission d'impulsions en réponse à la commande dudit élément pouvant être commande manuellement, fait également démarrer ledit temporisateur (29) en réponse à la commande dudit élément pouvant être commandé manuellement (28) et dans lequel lesdits moyens (29, 31) de déclenchement du second moyen d'engendrement et de transmission d'impulsions est le laps de temps sélectionné dudit temporisateur (29).

3. Appareil tel que revendiqué à la revendication 1, caractérisé en ce que chacun desdits premier (12–16, 18–23) et second (32–40, 43, 44) moyens d'engendrement et de transmission d'impulsions a la fonction d'engendrement et de transmission de sauts d'impulsions espacés dans le temps.

4. Appareil tel que revendiqué à la revendication 1, caractérisé en ce que ledit premier système de stimulation (24–27, 1f) comprend un second récepteur (25) et une électrode (1f) associée audit second récepteur, les deux électrodes dudit premier système de stimulation pouvant être positionnées sur des nerfs, toutes deux commandant la fonction dudit sphincter extérieur, et dans lequel ledit premier moyen d'engendrement et de transmission d'impulsions (12–16, 18–23) a la fonction d'engendrement et de transmission d'une série d'impulsions électriques aux deux récepteurs (24, 25) dudit premier système de stimulation.

5. Appareil tel que revendiqué à la revendication 4, caractérisé en ce que ledit premier moyen d'engendrement et de transmission d'impulsions (12–16, 18–23) a la fonction d'engendrement et de transmission de sauts d'impulsions espacés dans le temps séparément à chacun des deux récepteurs (24, 25) du premier système de stimulation.

6. Appareil tel que revendiqué à la revendication 5, caractérisé en ce que ledit premier moyen d'engendrement et de transmission d'impulsions (12–16, 18–23) a la fonction d'engendrement et de transmission de sauts d'impulsions alternativement aux deux récepteurs (24, 25) du premier système de stimulation.

7. Appareil tel que revendiqué à la revendication 1, caractérisé en ce que ledit second système de stimulation (45–48, 2f) comprend un second récepteur (46) et une électrode 2f associée audit second récepteur, les deux électrodes dudit second système de stimulation pouvant être positionnées sur des nerfs, toutes deux commandant la fonction de ladite vessie ou desdits intestins associés avec ledit sphincter extérieur et dans lequel ledit second moyen d'engendrement et de transmission d'impulsions (32–40, 43, 44) a la fonction d'engendrement et de transmission d'une série d'impulsions électriques aux deux récepteurs (45, 46) dudit second système de stimulation.

8. Appareil tel que revendiqué à la revendication 7, caractérisé en ce que ledit second moyen d'engendrement et de transmission d'impulsions (32–40, 43, 44) a la fonction d'engendrement et de transmission de sauts d'impulsions espacés dans le temps séparément à chacun des deux récepteurs (45, 46) du second système de stimulation.

9. Appareil tel que revendiqué à la revendication 8, caractérisé en ce que ledit second moyen d'engendrement et de transmission d'impulsions (32–40, 43, 44) a la fonction d'engendrement et de transmission de sauts d'impulsions alternativement aux deux récepteurs (45, 46) du second système de stimulation.

10. Appareil tel que revendiqué à la revendication 1, caractérisé en ce que ledit premier système de stimulation (24–27, 1f) comprend un second récepteur (25) et une électrode (1f) y associée, les deux électrodes dudit premier système de stimulation pouvant être positionnées sur des nerfs, toutes deux commandant la fonction dudit sphincter extérieur, dans lequel ledit second système de stimulation (45–48, 2f) comprend un second récepteur (46) et une électrode (2f) y associée, les deux électrodes dudit second système de stimulation pouvant être positionnées sur des nerfs, toutes deux commandant la fonction de ladite vessie ou desdits intestins avec ledit sphincter extérieur, dans lequel ledit premier moyen d'engendrement et de transmission d'impulsions (12–16, 18–23) a la fonction d'engendrement et de transmission d'une série d'impulsions électriques aux deux récepteurs (24, 25) dudit premier système de stimulation et dans lequel ledit second moyen d'engendrement et de transmission d'impulsions (32–40, 43, 44) a la fonction d'engendrement et de transmission d'une série d'impulsions électriques aux deux récepteurs (45, 46) dudit second système de stimulation.

11. Appareil tel que revendiqué à la revendication 10, caractérisé en ce que ledit premier moyen d'engendrement et de transmission d'impulsions (12–16, 18–23) a la fonction d'engendrement et de transmission de sauts d'impulsions espacés dans le temps séparément à chacun des deux récepteurs (24, 25) du premier système de stimulation et dans lequel ledit second moyen d'engendrement et de transmission d'impulsions (32–40, 43, 44) a la fonction d'engendrement et de transmission de sauts d'impulsions espacés dans le temps séparément à chacun des deux récepteurs (45, 46) du second système de stimulation.

12. Appareil tel que revendiqué à la revendication 10, caractérisé en ce que ledit premier moyen d'engendrement et de transmission d'impulsions (12–16, 18–23) a la fonction d'engendrement et de transmission de sauts d'impulsions alternativement aux deux récepteurs (24, 25) du premier système de stimulation et dans lequel ledit second moyen d'engendrement et de transmission d'impulsions (32–40, 43, 44) a la fonction d'engendrement et de transmission de sauts d'impulsions alternativement aux deux récepteurs (45, 46) du second système de stimulation.

# FIGURE 1

# FIGURE 2

FIGURE 3

FIGURE 4

FIGURE 5

FIGURE 6

FIGURE 7

FIGURE 8

FIGURE 9

FIGURE 10

FIGURE II

FIGURE 12

## FIGURE 13

## FIGURE 14

| | | |
|---|---|---|
| (28) SR STIM INITIATION PULSE | | |
| (29) SR TIMER | OFF   ON | 10 - 40 SEC |
| (30) SR DELAY | | 2 - 20 SEC |
| (31) SR OSC ENABLE | OFF   ON | OFF |
| (33) SR TRAIN OSC | RATE: 0.1-0.5 Hz | |
| (32) SR STIM PULSE OSC | RATE: 15-50 Hz | |
| (34) SR STIM PULSE | WIDTH 50-500 μs | |
| (35) SR STIM 1 | | |
| (36) SR STIM 2 | | |

| | | |
|---|---|---|
| (49) IS DELAY | | 2-20 SEC |
| (17) IS OSC ENABLE | ON | OFF   ON |
| (16) IS TRAIN OSC | RATE: 0.1-0.5 Hz | |
| (12) IS STIM PULSE OSC | RATE: 5-40 Hz | |
| (13) IS STIM PULSE | WIDTH: 50-500 μs | |
| (14) IS STIM 1 | | |
| (15) IS STIM 2 | | |

SR = SACRAL ROOT
IS = INFERIOR SOMATIC
OSC = OSCILLATOR

FIGURE 15

FIGURE 16

ON

OFF

63' { 63
64

65' { 65
66

EP 0 245 547 B1

FIGURE 17

FIGURE 18